# EUROPÄISCHE PATENTSCHRIFT

(11) **EP 3 747 377 B1**
(45) Veröffentlichungstag und Bekanntmachung des Hinweises auf die Patenterteilung: **30.11.2022**
(21) Anmeldenummer: 20176985.8
(22) Anmeldetag: 28.05.2020
(51) Int. Cl.: A61B 17/29, A61B 18/14

(54) **CHIRURGISCHES INSTRUMENT ZUR MINIMALINVASIVEN CHIRURGIE**
SURGICAL INSTRUMENT FOR MINIMALLY INVASIVE SURGERY
INSTRUMENT CHIRURGICAL DESTINÉ À LA CHIRURGIE MINI-INVASIVE

(30) Priorität: 04.06.2019 DE 102019115004
(43) Veröffentlichungstag der Anmeldung: 09.12.2020
(73) Patentinhaber: avateramedical GmbH, 07745 Jena (DE)
(72) Erfinder: Keim, Tobias, 07343 Wurzbach (DE); Noack, Stefan, 07743 Jena (DE)
(74) Vertreter: Schaumburg und Partner Patentanwälte mbB

(56) Entgegenhaltungen:
- EP-A1- 2 377 477
- EP-A1- 2 777 516
- US-A1- 2008 097 398
- US-A1- 2009 062 602
- US-A1- 2011 054 487

## Beschreibung

Die Erfindung betrifft ein chirurgisches Instrument zur minimalinvasiven Chirurgie, das einen Instrumentenschaft mit einem distalen Ende und einem proximalen Ende hat. Mit dem proximalen Ende ist ein Signalgeber verbindbar. Das chirurgische Instrument hat einen Instrumentenkopf, der über ein Scharnier schwenkbar mit dem distalen Ende des Instrumentenschafts verbunden ist. Ferner hat das chirurgische Instrument einen Endeffektor, der im Instrumentenkopf um seine Längsachse drehbar gelagert ist. Weiterhin umfasst das chirurgische Instrument mindestens ein mechanisches Kopplungselement, das zumindest teilweise im Instrumentenschaft angeordnet und zum Übertragen und/oder Übersetzen mechanischer Betätigungssignale des Signalgebers zum Schwenken und Drehen des Instrumentenkopfs ausgebildet ist. Eine Schraubenfeder überbrückt das Scharnier und verbindet den Endeffektor mit dem Kopplungselement, um den Endeffektor zu drehen. Ein solches Instrument zur minimalinvasiven Chirurgie ist beispielsweise aus dem Dokument EP 2 377 477 A1 bekannt.

Aus dem Dokument EP 3 025 667 A1 ist eine Vorrichtung zur robotergestützten Chirurgie bekannt, bei der ein chirurgisches Instrument mit Hilfe eines Manipulatorarms eines Manipulators geführt und über einen Signalgeber des Manipulatorarms betätigt wird. Hierzu ist am distalen Ende des Manipulatorarms eine Koppeleinheit mit Antriebselementen und Übertragungsmitteln angeordnet, mit der eine am distalen Ende eines Instrumentenschafts des chirurgischen Instruments angeordnete Instrumenteneinheit über eine Sterilschleuse koppelbar ist.

Bei dem aus dem Dokument EP 2 377 477 A1 bekannten chirurgischen Instrument ist die Schraubenfeder zum Überbrücken des Scharniers und zum Verbinden des Endeffektors mit dem Kopplungselement zum Drehen des Endeffektors derart ausgebildet und angeordnet, dass die Ganghöhe der Spiralfeder so eingestellt ist, dass bei maximaler Biegung der Spiralfeder entsprechend einer maximalen Verschwenkungsposition des Instrumentenkopfs Federwindungen an der bezüglich der Biegerichtung inneren Federseite gerade in Anlage kommen oder noch voneinander beabstandet sind. Dies hat insbesondere den Nachteil, das innenliegende Drähte, insbesondere Betätigungsdrähte oder elektrische Leiter z.B. zur Hochfrequenzchirurgie nicht sicher geführt sind, wenn die Windungen der Feder sich nicht oder nur im maximal abgewinkelten Zustand berühren.

Darüber hinaus ist es bei dem aus dem Stand der Technik bekannten Instrumenten nachteilig, dass die Feder im Wesentlichen an einer einzigen Stelle in einer zur Längsachse des Instrumentenschachts orthogonalen Ebene, in der auch die Drehachse des Scharniers verläuft, relativ stark abgewinkelt wird und die Windungen in diesem Bereich hoch beansprucht werden, wohingegen die angrenzenden weiteren Bereiche nicht oder wesentlich geringer gebogen werden.

Davon ausgehend ergibt sich die Aufgabe, ein chirurgisches Instrument zur minimalinvasiven Chirurgie anzugeben, das einfach aufgebaut ist und eine sichere Funktion gewährleistet.

Diese Aufgabe wird durch ein chirurgisches Instrument zur minimalinvasiven Chirurgie mit den Merkmalen des Anspruchs 1 gelöst. Vorteilhafte Weiterbildungen sind in den abhängigen Ansprüchen angegeben.

Durch ein chirurgisches Instrument zur minimalinvasiven Chirurgie mit den Merkmalen des Anspruchs 1 ist es möglich, das Biegeverhalten der Feder beim Abwinkein des Instrumentenkopfs zu beeinflussen. Insbesondere kann durch die Wahl geeigneter Federkonstanten in unterschiedlichen Bereichen der Schraubenfeder eine individuelle Anpassung der Biegeeigenschaften der Feder beim seitlichen Verschwenken des distalen Endes der Feder von der Längsachse des Instrumentenschafts weg erfolgen. Insbesondere kann die Form der Schraubenfeder nach dem Verschwenken des distalen Endes der Schraubenfeder von der Längsachse des Instrumentenschafts weg angepasst werden, vorzugsweise dahingehend, dass eine Hauptabwinklung mittig, d.h. im Bereich der Drehachse des Gelenks, erfolgt. Hierbei werden die Windungen im Bereich der Hauptabwinklung auf Grund einer vorzugsweise niedrigen Federkonstante weniger beansprucht als bei Schraubenfedern nach dem Stand der Technik. Mit Hilfe der unterschiedlichen Federkonstanten kann die Schraubenfeder aber auch derart konfiguriert werden, dass ein Bereich, indem die Hauptabwinklung der Feder erfolgt, einen Abstand zur Drehachse des mindestens einen Gelenks in proximaler Richtung oder in distaler Richtung hat. Besonders vorteilhaft ist es, wenn durch die Federkonstanten mindestens zwei, insbesondere drei oder vier, Hauptbiegestellen beim Abwinkeln des Instrumentenkopfs ausgebildet werden. Eine solche Konstruktion ist insbesondere für ein Gelenk, welches sich aus einer Vielzahl von Scharnieren zusammensetzt, vorteilhaft. Es ist auch möglich, mit Hilfe der unterschiedlichen Federkonstanten in Bereichen der Schraubenfeder einen gewünschten Biegeradius oder einen gewünschten Kurvenverlauf der Längsachse der Feder zu erzeugen. Hierdurch ist es möglich, dass eine Kraftübertragung zur Rotation des Instrumentenkopfs sicher und insbesondere kontinuierlich und ruckelfrei erfolgen kann. Jedoch kann das Gelenk auch als Festkörpergelenk, wie es aus dem Dokument WO 2006/113216 A2, bekannt ist, als einzelnes Scharnier, wie es aus dem Dokument EP 2 377 477 A1 bekannt ist, und/oder als Scharnierkette, wie sie aus dem Dokument EP 2 777 561 B1 bekannt ist, ausgebildet sein.

Wird im Inneren der Schraubenfeder mindestens ein Betätigungsdraht und/oder elektrisch leitender Draht hindurch geführt, können diese durch die einfach anpassbare Biegeform der Schraubenfeder auch beim Schwenken des Instrumentenkopf und dem damit verbundenen seitlichen Auslenken bzw. Verbiegen der Schraubenfeder sicher geführt werden, so dass Beschädigungen dieser Drähte vermieden werden.

Besonders vorteilhaft ist es, wenn die Federkonstante vom distalen Ende der Schraubenfeder und vom proximalen Ende der Schraubenfeder, vorzugsweise kontinuierlich, insbesondere gleichmäßig, zur Mitte oder zu einem mittleren Bereich mit konstanter Federkonstante der Schraubenfeder hin abnimmt oder zu diesem Bereich hin kontinuierlich, insbesondere gleichmäßig, zunimmt. Hierdurch kann eine Hauptabwinkelung in der Mitte der Feder erreicht werden.

Ferner ist es vorteilhaft, wenn die Windung der Schraubenfeder einen rechteckigen Querschnitt hat. Hierdurch können hohe Federkonstanten bei geringem Bauraum erreicht werden. Darüber hinaus erfolgt bei einer Übertragung einer Drehbewegung über die Schraubenfeder um die Längsachse der Schraubenfeder herum nur eine geringe elastische Verformung der Feder, so dass eine einfache Bedienbarkeit des Endeffektors möglich ist. Darüber hinaus lässt sich eine solche Schraubenfeder einfach durch mechanische Bearbeitungsverfahren, wie Fräsen und Wickeln, oder alternativ mit Hilfe eines 3D-Druckers herstellen.

Ferner ist es vorteilhaft, wenn die Windung der Schraubenfeder in den Bereichen mit unterschiedlicher Federkonstante eine unterschiedliche Querschnittsfläche hat. Durch die Änderung der Querschnittsfläche in Betrag und/oder der Form kann die Federkonstante in Bereichen der Schraubenfeder auch dann unterschiedlich sein, wenn die gesamte Schraubenfeder aus demselben Material hergestellt ist. Darüber hinaus lässt sich die Federkonstante durch Änderung der Querschnittsfläche auch in Verbindung mit weiteren Maßnahmen zur Ausbildung unterschiedlicher Federkonstanten in den Bereichen der Schraubenfeder kombinieren.

Ferner ist es vorteilhaft, wenn die Windung der Schraubenfeder in den Bereichen mit unterschiedlicher Federkonstante eine andere Steigung hat. Hierdurch ist eine einfache Herstellung einer Schraubenfeder mit Bereichen unterschiedlicher Federkonstanten möglich.

Ferner ist es vorteilhaft, wenn die Windung der Schraubenfeder Abschnitte mit Materialien mit unterschiedlichen Elastizitätsmodulen aufweist. Hierdurch kann die Federkonstante in den Bereichen einfach dadurch unterschiedlich erzeugt werden, dass die Bereiche der Schraubenfeder aus unterschiedlichen Materialien hergestellt sind. Dies ist insbesondere dann einfach möglich, wenn mehrere Federsegmente zu einer Gesamtschraubenfeder zusammengesetzt werden, indem die Windung der Bereiche miteinander verschweißt oder verklebt oder anderweitig verbunden werden. Alternativ können die unterschiedlichen Materialien auch nacheinander bei einem 3D-Druck zur Herstellung der Feder verwendet werden. Dabei ist auch ein kontinuierlicher Übergang zwischen verschiedenen Materialien oder Materialkonfigurationen denkbar.

Besonders vorteilhaft ist es, wenn die Schraubenfeder eine lineare Feder ist. Hierbei ist die Schraubenfeder vorzugsweise derart ausgebildet, dass sich die Feder bei Krafteinwirkung in Richtung der Mittelachse der Schraubenfeder mit zunehmender Kraft gleichmäßig, d.h. linear zur Kraft verformt. Hierbei erfolgt jedoch eine unterschiedliche Verformung in jedem der Bereiche mit unterschiedlicher Federkonstante.

Ferner ist es vorteilhaft, wenn das Kopplungselement einen Drehrohrschaft umfasst, wenn das proximale Ende der Schraubenfeder mit dem Drehrohrschaft drehfest verbunden ist und wenn der Drehrohrschaft drehbar im Instrumentenschaft gelagert ist. Hierdurch ist eine einfache und kompakte Anordnung der Bestandteile des Kopplungselements im Inneren des Instrumentenschafts möglich, wobei der Drehrohrschaft eine sichere, in Drehrichtung torsionsarme Antriebsmöglichkeit zum Drehen des Instrumentenkopfs bzw. des Endeffektors bietet.

Bei einer alternativen Ausführungsform ist die Schraubenfeder zwischen dem Drehrohrschaft und dem Instrumentenkopf vorgespannt angeordnet. Hierdurch können die Torsionseigenschaften der Schraubenfeder weiter angepasst werden. Ferner ist es vorteilhaft, wenn die Schraubenfeder derart ausgebildet und angeordnet ist, dass die Windungen der Schraubenfeder beim Verschwenken des Instrumentenkopfs ab einer vorbestimmten Schwenkposition an der bezüglich der Biegerichtung inneren Seite der Schraubenfeder in Anlage kommen und dass die Windungen der Schraubenfeder beim Verschwenken des Instrumentenkopfs über die vorbestimmte Schwenkposition hinaus bis zu einer maximalen Schwenkposition des Instrumentenkopfs an der bezüglich der Biegerichtung inneren Federseite mit einer Andruckkraft aufeinander drücken. Hierbei können die Windungen der Schraubenfeder in der maximalen Schwenkposition des Instrumentenkopfs an der äußeren Federseite einen größeren Abstand haben als in ihrer Ausgangslage mit geradem Instrumentenkopf, d.h. wenn der Instrumentenkopf nicht um die Drehachse des Gelenks verschwenkt ist. Durch den vergrößerten Abstand an der äußeren Federseite sind die Windungen dort zumindest in der maximalen Schwenkposition auseinander gezogen. Hierdurch wird sichergestellt, dass die Windungen an der Innenseite ab dem Berühren in der vorbestimmten Schwenkposition bis zur maximalen Schwenkposition immer aneinander liegen und im Inneren der Schraubenfeder geführten Drähte bzw. Züge an der Innenseite der Feder sicher und reibungsarm geführt sind, so dass Beschädigungen dieser Drähte vermieden werden.

Ferner ist es vorteilhaft, wenn der Instrumentenkopf mit Hilfe des Kopplungselements um eine Drehachse um einen vorbestimmten Winkel schwenkbar ist, wobei die Drehachse orthogonal zur Längsachse des Instrumentenschafts ist. Hierdurch ist eine einfache Ausbildung und Anordnung des Gelenks möglich. Das Gelenk kann hierbei als einfaches Scharnier ausgebildet sein. Hierbei ist es besonders vorteilhaft, wenn das Schwenken des Instrumentenkopfs durch eine Translationsbewegung eines zwischen Instrumentenschaft und Drehrohrschaft angeordneten Innenschafts entlang dessen Längsachse erfolgt. Hierdurch ist eine einfache und sichere Kopplung des Kopplungselements mit dem Instrumentenkopf möglich, um sowohl ein Verschwenken des gesamten Instrumentenkopfs als auch einer Rotationsbewegung des Endeffektors und/oder des Instrumentenkopfs sicher zu ermöglichen.

Besonders vorteilhaft ist es, wenn die Drehachse des Gelenks durch und orthogonal zur Längsachse der Schraubenfeder verläuft. Hierdurch ist ein einfacher und kompakter Aufbau des Instruments im Bereich des Instrumentenkopfs und des Übergangs zwischen Instrumentenschaft und Instrumentenkopf möglich.

Besonders vorteilhaft ist es, wenn die Schraubenfeder in Drehrichtung der Schraubenfeder zum Drehen des Instrumentenkopfs bzw. des Endeffektors bei üblichen zur Drehung des Instrumentenkopfs erforderlichen Kräften nicht oder nur gering verformt. Hierdurch ist eine einfache und sichere Betätigung des chirurgischen Instruments möglich. Insbesondere ist eine sichere Handhabung des chirurgischen Instruments gewährleistet, da eine gewünschte initiierte Drehbewegung relativ exakt über die Schraubenfeder übertragen wird.

Ferner ist es vorteilhaft, wenn der mit dem proximalen Ende des Instrumentenschafts verbundene Signalgeber eine manuelle Betätigungseinheit und/oder ein Koppelelement zum Koppeln des chirurgischen Elements mit mindestens einer Antriebseinheit eines Manipulatorarms eines Manipulators ist. Hierdurch kann eine einfache und sichere Bedienung des chirurgischen Instruments erfolgen.

Das chirurgische Instrument umfasst vorzugsweise mindestens einen in eine Körperöffnung eines Patienten einführbaren an Instrumentenkopf angeordneten Endeffektor, wie eine Klemme, eine Schere, einen Greifer, einen Nadelhalter, einen Mikrodissektor, eine Klammervorrichtung, eine Heftvorrichtung, eine Spül- und/oder Absaugvorrichtung, eine Schneidklinge, eine Kauterisationssonde, einen Katheter und/oder eine Saugdüse oder ein Endeffektor zur Hochfrequenzchirurgie. Dadurch kann das chirurgische Instrument wahlweise unterschiedliche Endeffektoren haben, die für gängige minimal-invasive Eingriffe, insbesondere bei der laparoskopischen Chirurgie, eingesetzt werden können. Es können jedoch auch andere chirurgische Instrumente zusätzlich oder alternativ eingesetzt werden.

Nachfolgend werden weitere Merkmale und Vorteile anhand von Ausführungsformen in Verbindung mit den beigefügten Figuren beschrieben.

Es zeigt:
- Figur 1: eine perspektivische Darstellung eines chirurgischen Instruments zur minimalinvasiven Chirurgie zum Einsatz mit einem Manipulator;
- Figur 2: eine Schnittdarstellung eines distalen Abschnitts des Instruments nach Figur 1;
- Figur 3: eine zum Übertragen einer Drehbewegung im Instrument nach den Figuren 1 und 2 eingesetzte Schraubenfeder;
- Figur 4: die Schraubenfeder nach Figur 3 in einem um 45° abgewinkelten Zustand;
- Figur 5: ein Diagramm mit dem Verlauf der Federkonstanten D über die Länge einer Schraubenfeder gemäß dem Stand der Technik;
- Figur 6: ein Diagramm mit dem Verlauf der Federkonstanten D über die Länge L der Schraubenfeder gemäß einer ersten Ausführungsform:
- Figur 7: ein Diagramm mit dem Verlauf der Federkonstanten D über die Länge L der Schraubenfeder gemäß einer zweiten Ausführungsform;
- Figur 8: ein Diagramm mit dem Verlauf der Federkonstanten D über die Länge L der Schraubenfeder gemäß einer dritten Ausführungsform;
- Figur 9: ein Diagramm mit dem Verlauf der Federkonstanten D über die Länge L der Schraubenfeder gemäß einer vierten Ausführungsform; und
- Figur 10: ein Diagramm mit dem Verlauf der Federkonstanten D über die Länge L der Schraubenfeder gemäß einer fünften Ausführungsform.

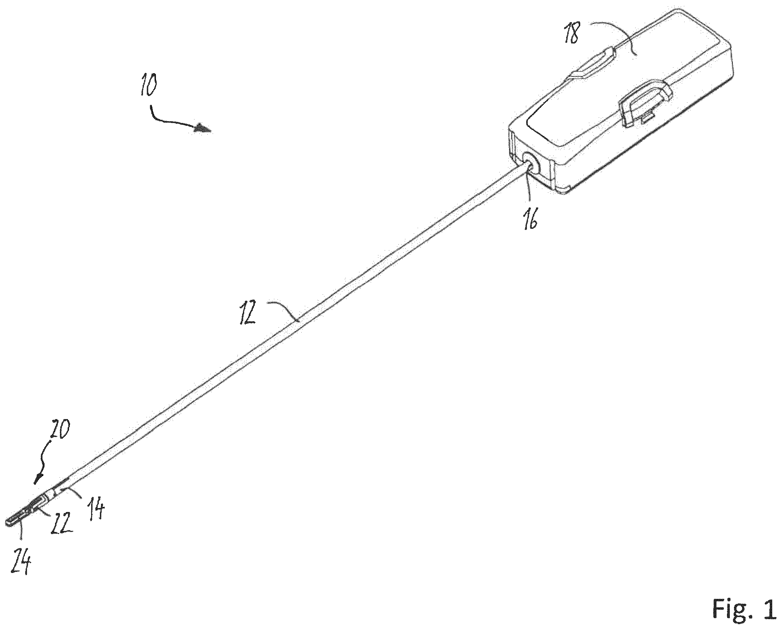

Figur 1 zeigt ein chirurgisches Instrument 10 zur minimalinvasiven Chirurgie. Dieses Instrument 10 hat einen Instrumentenschaft 12 mit einem distalen Ende 14 und einem proximalen Ende 16. Das proximale Ende 16 des Instrumentenschafts 12 ist mit einer Instrumenteneinheit 18 verbunden. Über die Instrumenteneinheit 18 ist das chirurgische Instrument 10 mit einer Koppeleinheit eines Manipulatorarms eines Manipulators verbindbar. Solche Manipulatoren werden auch als Telemanipulatorsysteme bezeichnet und dienen zur robotergestützten Chirurgie. Solche Manipulatoren sind insbesondere in den Dokumenten EP 3 025 667 A1 und WO 2016/083189 A1 offenbart. Die erfindungsgemäße Lösung kann ohne Änderungen auch für laparoskopische Handinstrumente verwendet werden.

Das chirurgische Instrument 10 hat einen mit dem distalen Ende 14 des Instrumentenschafts 12 über ein als Scharnier 22 ausgebildetes Gelenk verbundenen Instrumentenkopf 20. Der Instrumentenkopf 20 umfasst einen Endeffektor 24, der am Instrumentenkopf 20 um seine Längsachse drehbar gelagert ist.

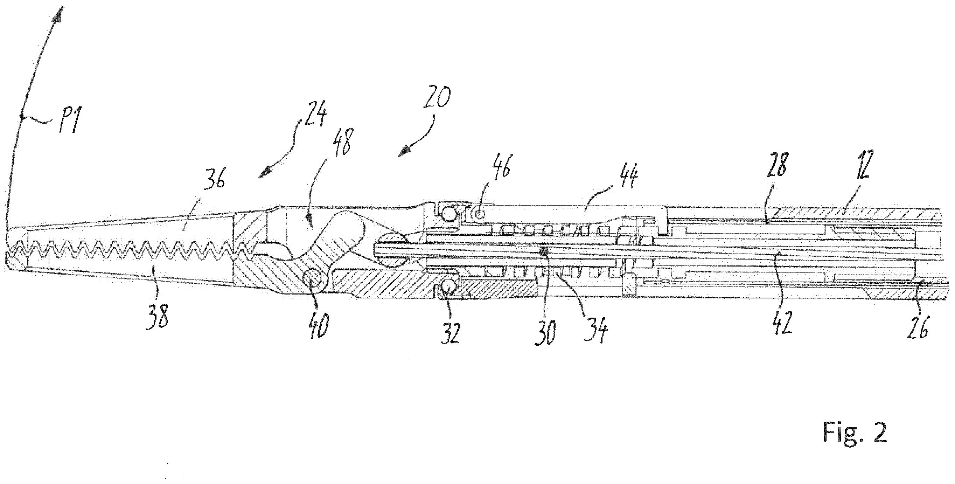

Figur 2 zeigt eine Schnittdarstellung eines distalen Abschnitts des chirurgischen Instruments 10 nach Figur 1. Im Inneren des Instrumentenschafts 12 ist ein drehbar zum Instrumentenschaft 12 gelagerter Drehrohrschaft 26 sowie ein zwischen dem Drehrohrschaft 26 und dem Instrumentenschaft 12 angeordneter Innenschaft 28 angeordnet, wobei der Innenschaft 28 zumindest in Längsrichtung des Instrumentenschafts 12 bewegt werden kann, um den Instrumentenkopf 20 um eine in Figur 2 zusätzlich eingezeichnete orthogonal zur Zeichnungsebene verlaufende Drehachse 30 in Richtung des Pfeils P1 zu verschwenken. Der Pfeil P1 gibt somit die Schwenkrichtung des Instrumentenkopfs 20 an, in die er aus der in Figur 2 gezeigten Ruhelage herausbewegt werden kann. Der maximale Winkel, um den der Instrumentenkopf 20 in Richtung des Pfeils P1 verschwenkt werden kann, liegt in der Regel in einem Bereich zwischen 60° und 90°, vorzugsweise bei 65° oder bei 70°. Bei anderen Ausführungsformen können anstatt des Scharniers 22 auch andere Gelenke eingesetzt werden.

Der Instrumentenkopf 20 ist über ein Kugellager 32 um die Längsachse des Instrumentenschafts 12 in der in Figur 2 gezeigten Ruhelage drehbar. Damit der Instrumentenkopf 20 auch bei einem um die Drehachse 30 verschwenkten Instrumentenkopf 20 gedreht werden kann, ist zur Übertragung der Drehbewegung eine Schraubenfeder 34 zwischen dem Drehrohrschaft 26 und dem Instrumentenkopf 20 vorgesehen, deren proximales Ende mit dem Drehrohrschaft 26 drehfest verbunden ist und deren distales Ende mit dem proximalen Ende des Instrumentenkopfs 20 drehfest verbunden ist. Die Schraubenfeder 34 ist im Inneren des Scharniers 22 angeordnet und überbrückt dieses. Der Endeffektor 24 hat ein erstes Maulteil 36 und ein zweites Maulteil 38. Das erste Maulteil 36 ist starr mit dem Instrumentenkopf 20 verbunden. Das zweite Maulteil ist um die durch den Stift 40 definierte Schwenkachse schwenkbar angeordnet, so dass der durch das erste Maulteil 36 und das zweite Maulteil 38 gebildete Greifer durch Verschwenken des zweiten Maulteils 38 um den Stift 40 geöffnet und geschlossen werden kann. Ein Verschwenken des zweiten Maulteils 38 um die durch den Stift 40 gebildete Drehachse wird durch einen Betätigungsdraht 42 und eine Betätigungsmechanik 48 gesteuert, wobei der Betätigungsdraht 42 zum Öffnen des Greifers eine Schubbewegung in Richtung des distalen Endes ausführt und zum Schließen des Greifers zum proximalen Ende hingezogen wird. Der Betätigungsdraht 42 verläuft im Inneren der Schraubenfeder 34 durch diese hindurch, so dass auch bei verschwenktem Instrumentenkopf 20 eine Betätigung des Endeffektors 24 bzw. eine Betätigung des Greifers möglich ist. In anderen Ausführungsformen ist das zweite Maulteil 38 ebenfalls beweglich ausgestaltet.

Bei dem Endeffektor 24 oder bei anderen Endeffektoren kann über den Betätigungsdraht 42 zusätzlich eine elektrische Verbindung zum Endeffektor 24 hergestellt werden, um den Endeffektor 24 und das chirurgische Instrument 10 zur Hochfrequenzchirurgie einzusetzen.

Der Innenschaft 28 steht mit dem proximalen Ende eines Zughebels 44 in Verbindung. Der Zughebel 44 ist um eine Schwenkachse 46 schwenkbar am Instrumentenkopf 20 angelenkt. Durch eine durch den Innenschaft 28 initiierte Bewegung des proximalen Endes des Zughebels 44 in Richtung des proximalen Endes des Instrumentenschafts 12 wird der Instrumentenkopf um die Drehachse 30 des Scharniers 22 verschwenkt und die Schraubenfeder 34 seitlich gebogen.

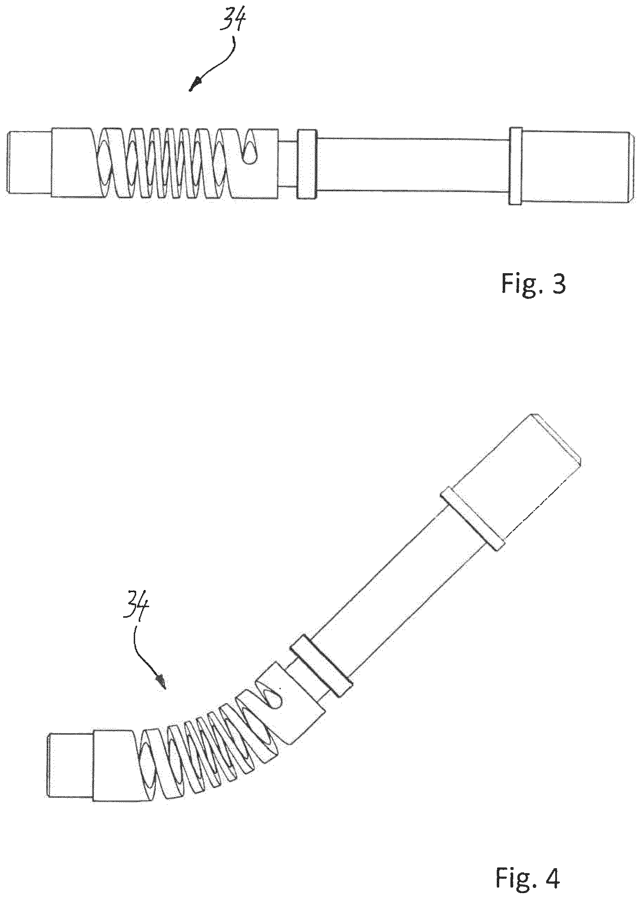

Figur 3 zeigt die Feder 24 nach Figur 2 in einem vorgespannten Zustand, mit dem die Schraubenfeder 34 in dem chirurgischen Instrument 10 angeordnet ist. Die Schraubenfeder 34 ist aus einem Federstahl gefertigt, wobei die Querschnittsfläche der Windungen vom proximalen Ende der Schraubenfeder 34 und vom distalen Ende der Schraubenfeder 34 zur Mitte der Schraubenfeder 34 hin abnimmt, so dass die Federkonstante jeweils vom proximalen und vom distalen Ende der Schraubenfeder 34 her abnimmt. Die Steigung der Schraubenfeder 34 im entspannten Zustand und/oder vorgespannten Zustand ist vorzugsweise konstant. Bei anderen Ausführungsformen kann zusätzlich oder alternativ zur Beeinflussung und Änderung der Federkonstante über die Länge der Schraubenfeder 34 hinweg die Steigung der Windungen unterschiedlich sein oder die Federeigenschaften des Materials in Abschnitten der Windungen unterschiedlich sein. Dies kann insbesondere durch das Zusammensetzen der Schraubenfeder 34 aus verschiedenen Windungsabschnitten erfolgen. Alternativ oder zusätzlich können einzelne Windungsabschnitte partiell gehärtet werden.

Figur 4 zeigt die Feder nach Figur 3 in einem Zustand nach dem Verschwenken des Instrumentenkopfs 20 relativ zum Instrumentenschaft 28 um einen Winkel von ca. 45°, wobei eine Längsachse des Instrumentenkopfs und die Längsachse des Instrumentenschafts einen Winkel von 135° aufspannen.

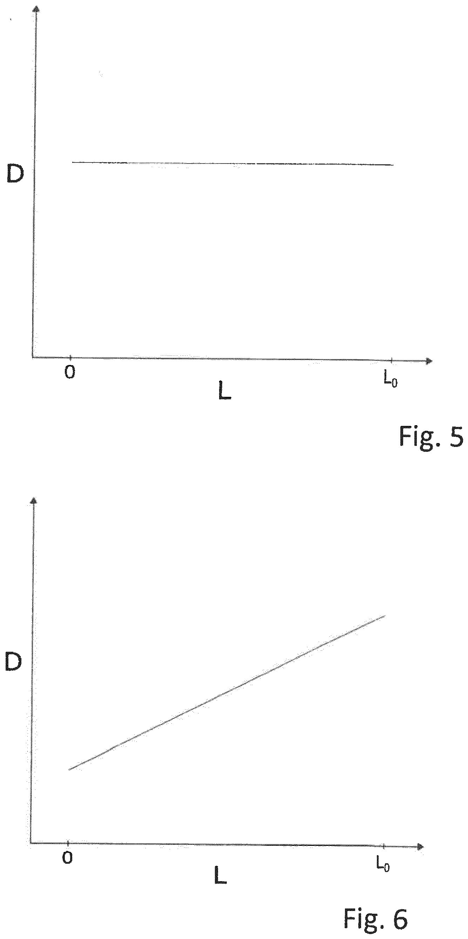

Figur 5 zeigt den Verlauf der Federkonstante D über die Länge L einer Schraubenfeder, wobei Null das proximale Ende der Schraubenfeder und L0 das distale Ende der Schraubenfeder ist. Bei der Schraubenfeder nach Figur 5 handelt es sich um eine Schraubenfeder mit konstanter Federkonstante D, wie sie bei chirurgischen Instrumenten im Stand der Technik eingesetzt wird.

Figur 6 zeigt den Verlauf der Federkonstante D über die Länge der Schraubenfeder 34 gemäß einer ersten Ausführungsform. Hierbei nimmt die Federkonstante D vom proximalen Ende der Schraubenfeder 34 zum distalen Ende der Schraubenfeder 34 kontinuierlich zu. Dies kann z.B. dadurch geschehen, dass der Querschnitt der Windungen ebenfalls vom proximalen Ende zum distalen Ende der Schraubenfeder 34 hin kontinuierlich zunimmt.

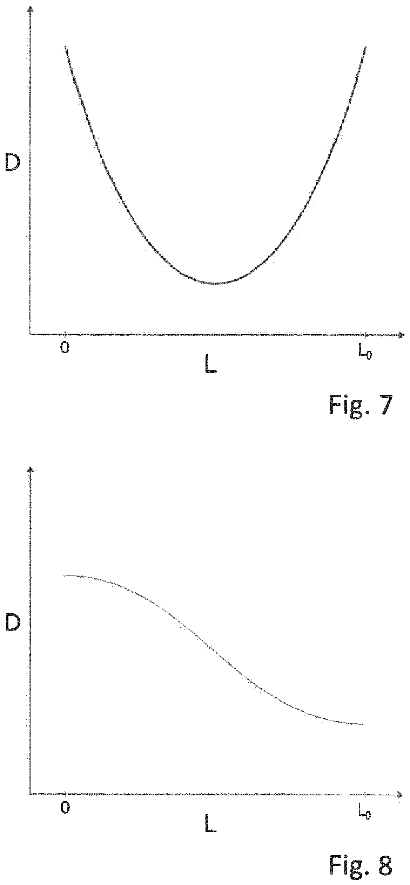

Figur 7 zeigt einen Verlauf der Federkonstante D über die Länge L der Schraubenfeder 34 gemäß einer zweiten Ausführungsform. Der in Figur 7 gezeigte Verlauf der Federkonstante D ist der Verlauf der durch die Querschnittsveränderung der Schraubenfeder 34 nach den Figuren 2 bis 4 bewirkten Federkonstante D, wenn die Schraubenfeder 34 eine konstante Steigung bzw. konstante Ganghöhe und über die gesamte Länge der Schraubenfeder 34 konstante Materialeigenschaften hat.

Figur 8 zeigt den Verlauf der Federkonstante D über die Länge einer Schraubenfeder 34 gemäß einer dritten Ausführungsform. Hierbei nimmt die Federkonstante vom proximalen Ende zum distalen Ende hin nicht linear ab.

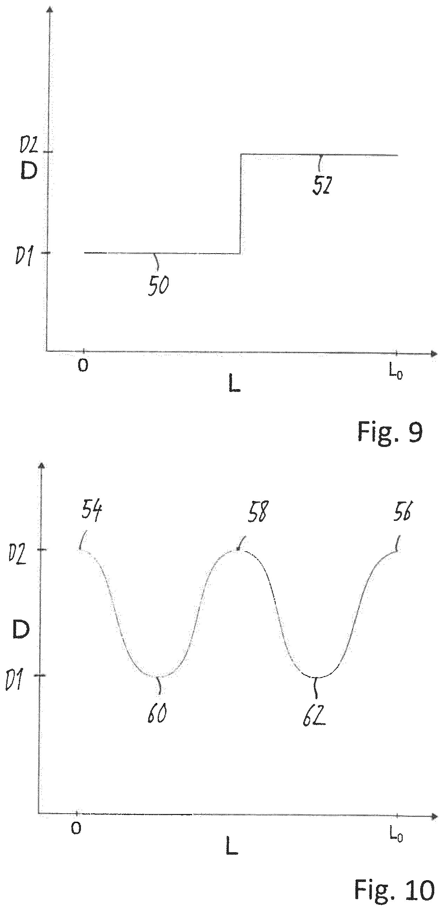

Figur 9 zeigt den Verlauf der Federkonstante D über die Länge L der Schraubenfeder 34 gemäß einer vierten Ausführungsform. Die Schraubenfeder 34 hat einen ersten Bereich 50 mit einer konstanten Federkonstante D1 und einen zweiten Bereich 52 mit einer konstanten Federkonstante D2. Ein solcher Verlauf der Federkonstante D wird beispielsweise durch eine stufenförmige Querschnittsänderung der Windung der Schraubenfeder 34 und/oder durch unterschiedliche Materialien in den beiden Bereichen 50, 52 mit konstanter Federkonstante D1, D2 erreicht, wobei die beiden Bereiche 50, 52 mit unterschiedlichen Materialien in der Mitte der Schraubenfeder 34, d.h. an dem stufenförmigen Übergang zwischen niedriger Federkonstante D1 und hoher Federkonstante D2 miteinander verbunden sind.

Figur 10 zeigt den Verlauf der Federkonstante D über die Länge einer Schraubenfeder 34 gemäß einer fünften Ausführungsform. Bei dem gezeigten Verlauf ist die Federkonstante D der Schraubenfeder 34 an dem distalen Ende 54 und proximalen Ende 56 sowie in der Mitte 58 hoch, wobei zwischen dem distalen Ende 54 und der Mitte 58 und zwischen der Mitte 58 und dem proximalen Ende 56 jeweils ein Minimum 60, 62 der Federkonstante D erreicht ist. Der in Figur 10 gezeigte Verlauf hat zur Folge, dass in den Bereichen 60, 62 mit minimaler Federkonstante D eine große Biegung beim Verschwenken des Instrumentenkopfs 20 erfolgt und in den Bereichen 54, 56, 58 mit hoher Federkonstante eine geringere Verformung.

Wie in den Figuren 6 bis 10 gezeigt, kann durch die Bereiche mit unterschiedlicher Federkonstante eine individuelle Anpassung der Torsions- bzw. Biegeeigenschaften der Schraubenfeder 34 beim Verschwenken des Instrumentenkopfs 20 erreicht werden. Insbesondere kann die Federkonstante D über die Länge der Schraubenfeder 34 so verändert werden, dass der Hauptbiegebereich mittig, d.h. im Bereich der Drehachse 30 des Scharniers 22, nicht mittig, d.h. vor und/oder nach der Drehachse 30 des Scharniers 22 erfolgen, wobei eine, zwei, drei oder vier Biegestellen vorgesehen werden können. Durch eine gegenseitige Berührung der Windungen der Schraubenfeder 34 beim Verschwenken des Instrumentenkopfs 20 erfolgt eine Stabilisierung der innenliegenden Drähte 42.

Schraubenfedern 34 mit Bereichen mit unterschiedlicher Federkonstante D können insbesondere durch Wickeln, Spanen, Laserschneiden, 3D-Druck, Schweißen, Kleben und andere geeignete Herstellungsverfahren hergestellt werden. Die Variation der Federkonstante D über die Länge L der Schraubenfeder 34 erfolgt insbesondere durch eine Änderung des Querschnitts der Windung, durch eine unterschiedliche Steigung der Windung der Schraubenfeder 34, durch unterschiedliche Materialien und/oder Materialgradienten, durch partielles Härten oder andere geeignete Wahl der Federkonfiguration. Durch die geeignete Variation der Federkonstante D über die Länge der Schraubenfeder 34 kann insbesondere die Torsionssteifigkeit der Schraubenfeder 34 und das Verhalten der Schraubenfeder 34 beim Abwinkeln des Instrumentenkopfs 20 beeinflusst werden.

### Bezugszeichenliste

- 10: chirurgisches Instrument
- 12: Instrumentenschaft
- 14: distales Ende
- 16: proximales Ende
- 18: Instrumenteneinheit
- 20: Instrumentenkopf
- 22: Scharnier
- 24: Endeffektor
- 26: Drehrohrschaft
- 28: Innenschaft
- 30: Drehachse
- 32: Kugellager
- 34: Schraubenfeder
- 36: festes Maulteil
- 38: bewegbares Maulteil
- 40: Stift, Schwenkachse
- 42: Betätigungsdraht
- 44: Zughebel
- 46: Schwenkachse
- 48: Betätigungsmechanik
- 50 bis 62: Bereich
- P1: Schwenkrichtung

## Patentansprüche

1. Chirurgisches Instrument zur minimalinvasiven Chirurgie,
mit einem Instrumentenschaft (12) mit einem distalen Ende (14) und einem proximalen Ende (16), mit dem ein Signalgeber (18) verbindbar ist,
mit einem Instrumentenkopf (20), der über mindestens ein Gelenk (22) schwenkbar mit dem distalen Ende des Instrumentenschafts (12) verbunden ist,
mit einem Endeffektor (24), der im Instrumentenkopf (20) um seine Längsachse drehbar gelagert ist,
mit mindestens einem mechanischen Kopplungselement (26, 28, 42), das zumindest teilweise im Instrumentenschaft (12) angeordnet und zum Übertragen und/oder Übersetzen mechanischer Betätigungssignale des Signalgebers (18) zum Schwenken und Drehen des Instrumentenkopfs (20) ausgebildet ist,
eine Schraubenfeder (34), die das Gelenk (22) überbrückt und den Endeffektor (24) mit dem Kopplungselement (26, 28, 42) verbindet, um den Endeffektor (24) zu drehen, **dadurch gekennzeichnet,**
**dass** die Windung der Schraubenfeder (34) Bereiche (50 bis 62) mit unterschiedlichen Federkonstanten (D) hat.

2. Vorrichtung nach Anspruch 1, **dadurch gekennzeichnet, dass** die Federkonstante vom distalen Ende der Schraubenfeder (34) und vom proximalen Ende der Schraubenfeder (34), vorzugsweise kontinuierlich, insbesondere gleichmäßig, zur Mitte oder zu einem mittleren Bereich mit konstanter Federkonstante (D) der Schraubenfeder (34) hin abnimmt.

3. Vorrichtung nach einem der vorhergehenden Ansprüche, **dadurch gekennzeichnet, dass** die Windung der Schraubenfeder (34) einen rechteckigen Querschnitt hat.

4. Vorrichtung nach einem der vorhergehenden Ansprüche, **dadurch gekennzeichnet, dass** die Windung der Schraubenfeder (34) in den Bereichen mit unterschiedlicher Federkonstante (D) unterschiedliche Querschnittsflächen hat.

5. Vorrichtung nach einem der vorhergehenden Ansprüche, **dadurch gekennzeichnet, dass** die Windung der Schraubenfeder (34) in den Bereichen mit unterschiedlicher Federkonstante (D) andere Steigungen hat.

6. Vorrichtung nach einem der vorhergehenden Ansprüche, **dadurch gekennzeichnet, dass** die Windung der Schraubenfeder (34) in den Bereichen mit unterschiedlicher Federkonstante (D) Materialien mit unterschiedlichen Elastizitätsmodulen aufweist.

7. Vorrichtung nach einem der vorhergehenden Ansprüche, **dadurch gekennzeichnet, dass** die Schraubenfeder (34) eine lineare Feder ist.

8. Vorrichtung nach einem der vorhergehenden Ansprüche, **dadurch gekennzeichnet, dass** das Kopplungselement (26, 28, 42) einen Drehrohrschaft (26) umfasst, dass das proximale Ende der Schraubenfeder (34) mit dem Drehrohrschaft (26) drehfest verbunden ist, und dass der Drehrohrschaft (26) drehbar im Instrumentenschaft (12) gelagert ist.

9. Vorrichtung nach Anspruch 8, **dadurch gekennzeichnet, dass** die Schraubenfeder (34) im zwischen Drehrohrschaft (26) und Instrumentenkopf (20) vorgespannt angeordnet ist.

10. Vorrichtung nach einem der vorhergehenden Ansprüche, **dadurch gekennzeichnet, dass** die Schraubenfeder (34) derart ausgebildet und angeordnet ist, dass die Windungen der Schraubenfeder (34) beim Schwenken des Instrumentenkopfs (20) ab einer vorbestimmten Schwenkposition an der bezüglich der Biegerichtung inneren Seite der Schraubenfeder (34) in Anlage kommen, und dass die Windungen der Schraubenfeder (34) beim Schwenken des Instrumentenkopfs (20) in eine maximale Schwenkposition des Instrumentenkopfs (20) an der bezüglich der Biegerichtung inneren Federseite mit einer Andruckkraft aufeinander drücken, wobei an der äußeren Federseite der Abstand der Windungen untereinander gegenüber einer Ausgangslage vergrößert ist.

11. Vorrichtung nach einem der vorhergehenden Ansprüche, **dadurch gekennzeichnet, dass** der Instrumentenkopf (20) mit Hilfe des Kopplungselements (26, 28, 42) um eine Drehachse (30) um einen vorbestimmten Winkel schwenkbar ist, wobei die Drehachse (30) orthogonal zur Längsachse des Instrumentenschafts (12) ist.

12. Vorrichtung nach Anspruch 11, **dadurch gekennzeichnet, dass** das Schwenken des Instrumentenkopfs (20) durch eine Translationsbewegung eines zwischen Instrumentenschaft (12) und Drehrohrschaft (26) angeordneten Innenschafts (28) entlang dessen Längsachse erfolgt.

13. Vorrichtung nach Anspruch 11 oder 12, **dadurch gekennzeichnet, dass** die Drehachse (30) des Gelenks (22) durch und/oder orthogonal zur Längsachse der Schraubenfeder (34) und/oder in einer zur Längsachse orthogonalen Ebene der Schraubenfeder (34) verläuft.

14. Vorrichtung nach einem der vorhergehenden Ansprüche, **dadurch gekennzeichnet, dass** die Schraubenfeder (34) torsionssteif ausgebildet ist.

15. Vorrichtung nach einem der vorhergehenden Ansprüche, **dadurch gekennzeichnet, dass** der mit dem proximalen Ende (16) des Instrumentenschafts (12) verbundene Signalgeber (18) eine manuelle Betätigungseinheit und/oder ein Koppelelement zum Koppeln des chirurgischen Instruments (10) mit mindestens einer Antriebseinheit eines Manipulatorarms eines Manipulators ist.

## Claims

1. A surgical instrument for minimally invasive surgery,
with an instrument shaft (12) with a distal end (14) and a proximal end (16) to which a signal generator (18) is connectable,
with an instrument head (20) which is pivotably connected to the distal end of the instrument shaft (12) via at least one joint (22),
with an end effector (24) which is mounted rotatably about its longitudinal axis in the instrument head (20),
with at least one mechanical coupling element (26, 28, 42) which is arranged at least partially in the instrument shaft (12) and is configured to transmit and/or convert mechanical actuating signals of the signal generator (18) for pivoting and rotating the instrument head (20),
a coil spring (34) which bypasses the joint (22) and connects the end effector (24) to the coupling element (26, 28, 42) to rotate the end effector (24), **characterized in that**
the coil of the coil spring (34) has portions (50 to 62) with different spring constants (D).

2. The device according to claim 1, **characterized in that** the spring constant decreases from the distal end of the coil spring (34) and from the proximal end of the coil spring (34), preferably continuously, in particular uniformly, toward the center or to a central area with a constant spring constant (D) of the coil spring (34).

3. The device according to one of the preceding claims, **characterized in that** the coil of the coil spring (34) has a rectangular cross-section.

4. The device according to one of the preceding claims, **characterized in that** the coil of the coil spring (34) has different cross-sectional areas in the portions with different spring constants (D).

5. The device according to one of the preceding claims, **characterized in that** the coil of the coil spring (34) has different pitches in the portions with different spring constants (D).

6. The device according to one of the preceding claims, **characterized in that** the coil of the coil spring (34) has materials with different moduli of elasticity in the portions with different spring constants (D).

7. The device according to one of the preceding claims, **characterized in that** the coil spring (34) is a linear spring.

8. The device according to one of the preceding claims, **characterized in that** the coupling element (26, 28, 42) comprises a rotating tube shaft (26), that the proximal end of the coil spring (34) is connected to the rotating tube shaft (26) in a rotationally fixed manner, and that the rotating tube shaft (26) is mounted rotatably in the instrument shaft (12).

9. The device according to claim 8, **characterized in that** the coil spring (34) is arranged between the rotating tube shaft (26) and the instrument head (20) in a biased manner.

10. The device according to one of the preceding claims, **characterized in that** the coil spring (34) is configured and arranged such that the coils of the coil spring (34), from a predetermined pivot position of the instrument head when pivoting the instrument head (20), come into contact at the inner side of the coil spring (34) with respect to the bending direction, and that the coils of the coil spring (34) when pivoting the instrument head (20) into a maximum pivot position of the instrument head (20) press against each other with a press-on force on the inner side of the spring with respect to the bending direction, wherein on the outer side of the spring the distance of the coils to each other is increased as compared to an initial position.

11. The device according to one of the preceding claims, **characterized in that** the instrument head (20) is pivotable about an axis of rotation (30) by a predetermined angle with the aid of the coupling element (26, 28, 42), wherein the axis of rotation (30) is orthogonal to the longitudinal axis of the instrument shaft (12).

12. The device according to claim 11, **characterized in that** the pivoting of the instrument head (20) is caused by a translatory motion of an inner shaft (28) along its longitudinal axis, which inner shaft is arranged between the instrument shaft (12) and the rotating tube shaft (26).

13. The device according to claim 11 or 12, **characterized in that** the axis of rotation (30) of the joint (22) runs through and/or orthogonal to the longitudinal axis of the coil spring (34) and/or in a plane of the coil spring (34) that is orthogonal to the longitudinal axis.

14. The device according to one of the preceding claims, **characterized in that** the coil spring (34) is designed in a torsionally stiff manner.

15. The device according to one of the preceding claims, **characterized in that** the signal generator (18) connected to the proximal end (16) of the instrument shaft (12) is a manual actuating unit and/or a coupling element for coupling the surgical instrument (10) to at least one drive unit of a manipulator arm of a manipulator.

## Revendications

1. Instrument chirurgical destiné à une chirurgie mini-invasive, comprenant
une tige d'instrument (12) avec une extrémité distale (14) et une extrémité proximale (16), à laquelle peut être connecté un émetteur de signal (18),
une tête d'instrument (20) qui est connectée de manière pivotante à l'extrémité distale de la tige d'instrument (12) par l'intermédiaire d'au moins une articulation (22),
un effecteur d'extrémité (24) qui est monté dans la tête d'instrument (20) pouvant tourner autour de son axe longitudinal,
au moins un élément de couplage mécanique (26, 28, 42), qui est agencé au moins partiellement dans la tige d'instrument (12) et qui est conçu pour transmettre et/ou traduire des signaux d'actionnement mécanique de l'émetteur de signal (18) afin de faire pivoter et tourner la tête d'instrument (20),
un ressort hélicoïdal (34) qui enjambe l'articulation (22) et connecte l'effecteur d'extrémité (24) à l'élément de couplage (26, 28, 42) pour faire tourner l'effecteur d'extrémité (24), **caractérisé en ce que** :
l'enroulement du ressort hélicoïdal (34) présente des zones (50 à 62) avec des constantes de ressort différentes (D).

2. Dispositif selon la revendication 1, **caractérisé en ce que** les constantes de ressort diminuent depuis l'extrémité distale du ressort hélicoïdal (34) et depuis l'extrémité proximale du ressort hélicoïdal (34), de préférence de manière continue, en particulier uniforme, vers le centre ou vers une zone centrale avec une constante de ressort (D) constante du ressort hélicoïdal (34).

3. Dispositif selon l'une quelconque des revendications précédentes, **caractérisé en ce que** l'enroulement du ressort hélicoïdal (34) présente une section transversale rectangulaire.

4. Dispositif selon l'une quelconque des revendications précédentes, **caractérisé en ce que** l'enroulement du ressort hélicoïdal (34) présente des surfaces de section transversale différentes dans les zones avec une constante de ressort différente (D).

5. Dispositif selon l'une quelconque des revendications précédentes, **caractérisé en ce que** l'enroulement du ressort hélicoïdal (34) présente d'autres pentes dans les zones avec une constante de ressort différente (D).

6. Dispositif selon l'une quelconque des revendications précédentes, **caractérisé en ce que** l'enroulement du ressort hélicoïdal (34) présente, dans les zones avec une constante de ressort différente (D), des matériaux comprenant des modules d'élasticité différents.

7. Dispositif selon l'une quelconque des revendications précédentes, **caractérisé en ce que** le ressort hélicoïdal (34) est un ressort linéaire.

8. Dispositif selon l'une quelconque des revendications précédentes, **caractérisé en ce que** l'élément de couplage (26, 28, 42) comprend une tige tubulaire rotative (26), **en ce que** l'extrémité proximale du ressort hélicoïdal (34) est reliée de manière solidaire en rotation à la tige tubulaire rotative (26), et **en ce que** la tige tubulaire rotative (26) est montée de manière rotative dans la tige d'instrument (12).

9. Dispositif selon la revendication 8, **caractérisé en ce que** le ressort hélicoïdal (34) est agencé en précontrainte entre la tige tubulaire rotative (26) et la tête d'instrument (20).

10. Dispositif selon l'une quelconque des revendications précédentes, **caractérisé en ce que** le ressort hélicoïdal (34) est conçu et agencé de sorte que les enroulements du ressort hélicoïdal (34) viennent en appui sur le côté intérieur du ressort hélicoïdal (34) par rapport à la direction de flexion lors du pivotement de la tête d'instrument (20) à partir d'une position de pivotement prédéterminée, et **en ce que** les enroulements du ressort hélicoïdal (34) sont pressés mutuellement avec une force de pression sur le côté intérieur du ressort par rapport à la direction de flexion lors du pivotement de la tête d'instrument (20) dans une position de pivotement maximum, dans lequel la distance entre les enroulements est augmentée au niveau du côté extérieur du ressort par rapport à une position de départ.

11. Dispositif selon l'une quelconque des revendications précédentes, **caractérisé en ce que** la tête d'instrument (20) peut être pivotée d'un angle prédéterminé autour d'un axe de rotation (30) à l'aide de l'élément de couplage (26, 28, 42), dans lequel l'axe de rotation (30) est orthogonal à l'axe longitudinal de la tige d'instrument (12).

12. Dispositif selon la revendication 11, **caractérisé en ce que** le pivotement de la tête d'instrument (20) s'effectue par un déplacement en translation d'une tige intérieure (28) agencée entre la tige d'instrument (12) et la tige tubulaire rotative (26) le long de son axe longitudinal.

13. Dispositif selon la revendication 11 ou 12, **caractérisé en ce que** l'axe de rotation (30) de l'articulation (22) s'étend à travers et/ou orthogonalement à l'axe longitudinal du ressort hélicoïdal (34) et/ou dans un plan orthogonal à l'axe longitudinal du ressort hélicoïdal (34).

14. Dispositif selon l'une quelconque des revendications précédentes, **caractérisé en ce que** le ressort hélicoïdal (34) est formé rigide en torsion.

15. Dispositif selon l'une quelconque des revendications précédentes, **caractérisé en ce que** l'émetteur de signal (18) relié à l'extrémité proximale (16) de la tige d'instrument (12) est une unité à actionnement manuel et/ou un élément de couplage pour coupler l'instrument chirurgical (10) à au moins une unité d'entraînement d'un bras manipulateur d'un manipulateur.
